# EUROPEAN PATENT SPECIFICATION

(11) **EP 1 654 271 B1**
(45) Date of publication and mention of the grant of the patent: **10.12.2008**
(21) Application number: 04740827.3
(22) Date of filing: 08.07.2004
(51) Int. Cl.: C07J 71/00, A61K 31/56

(54) **STEROID MODIFIED SOLATRIOSES**
STEROID MODIFIZIERTE SOLATRIOSEN
SOLATRIOSES A MODIFICATION STEROIDE

(30) Priority: 08.07.2003 EP 03015502
(43) Date of publication of application: 10.05.2006
(73) Proprietor: GlycoMed Sciences Limited, George Town, Grand Cayman (KY)
(72) Inventor: Lawson, Christopher John, Dextra Laboratories, Ltd, Reading RG6 6BZ (GB); Weymouth-Wilson, Alexander Charles, Reading RG6 6BZ (GB)
(74) Representative: Vossius & Partner
(86) International application number: PCT/EP2004/007537
(87) International publication number: WO 2005/005454

(56) References cited:
- WO-A-03/018604
- WO-A-20/04096830
- LI B ET AL: "An improved synthesis of the saponin, polyphyllin D" CARBOHYDRATE RESEARCH, ELSEVIER SCIENTIFIC PUBLISHING COMPANY. AMSTERDAM, NL, vol. 331, no. 1, 9 March 2001 (2001-03-09), pages 1-7, XP004317141 ISSN: 0008-6215
- BITE P ET AL: "PREPARATION OF STEROID GLYCOSIDES" ACTA CHIMICA ACADEMIAE SCIENTIARUM HUNGARICA, BUDAPEST, HU, vol. 52, no. 1, 1967, pages 79-82, XP001127494 ISSN: 0001-5407
- PACZKOWSKI C ET AL: "GLUCOSYLATION AND GALACTOSYLATION OF DIOSGENIN AND SOLASODINE BY SOLUBLE GLYCOSYLTRANSFERASE(S) FROM SOLANUM MELONGENA LEAVES" PHYTOCHEMISTRY, PERGAMON PRESS, GB, vol. 35, no. 6, 19 April 1994 (1994-04-19), pages 1429-1434, XP001132072 ISSN: 0031-9422
- MIN-JWEI SHIH ET AL.: "alpha- and beta-Solamarine in Kennebec Solanum Tuberosum Leaves and aged Tuber Slices" PHYTOCHEMISTRY, vol. 13, 1974, pages 997-1000, XP002308073

## Description

The present invention relates to the chemical synthesis of alkaloid glycosides, in particular to the synthesis of steroid modified solatrioses. Furthermore, the present invention relates to intermediate compounds useful for the preparation of steroid modified solatrioses.

The aglycon solasodine is a source for synthetic cortisone and progesterone. Solasodine and its glycosides are of considerable interest commercially and clinically. They are widely used as starting products for the synthesis of various steroidal drugs.

It is moreover well established that certain naturally occurring conjugate solasodine glycosides have potent antineoplastic properties. Of particular interest is the chacotriose type triglycoside solamargine (22R, 25R)-spiro-5-en-3β-yl-α-L-rhamnopyranosyl-(1->2 glu)-α-L-rhamnopyranosyl- (1->4 glu)-β-D-gluco-pyranose. The structure of this triglycoside is as follows:

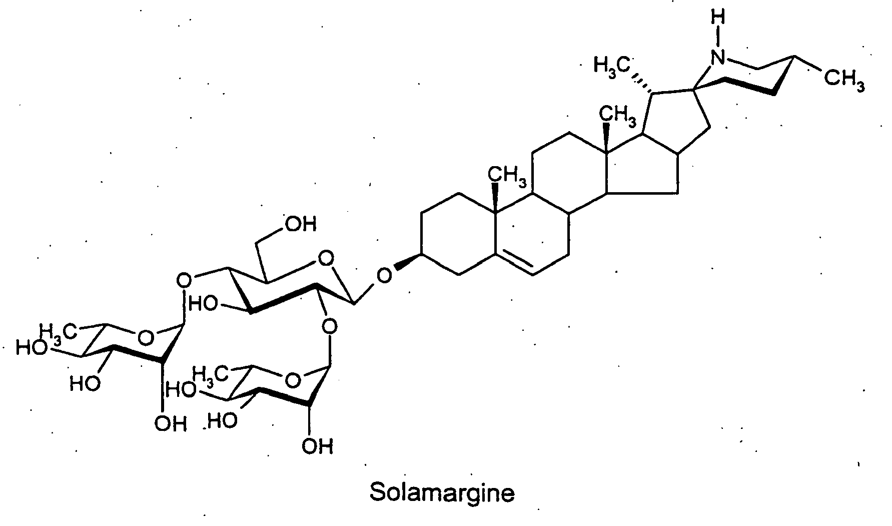

Another naturally occurring conjugate solasodine glycoside of particular interest is the solatriose type triglycoside solasonine (22R, 25R)-spiro-5-en-3β-yl-α-L-rhamno-pyranosyl-(1->2 gal)-O-p-D-glucopyranosyl-(1->3 gal)-β-D-galactopyranose. The structure of this triglycoside is as follows:

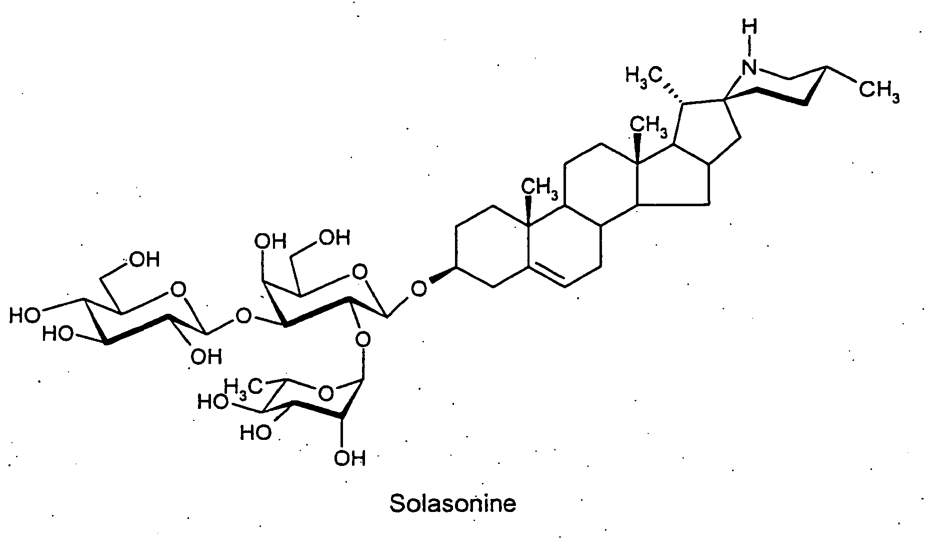

The above triglycosides are conventionally obtained by extraction from a plant source. A commercially available extract of *S*. *sodomaeum*, commonly referred to as BEC (Drug Future, 1988, vol. 13.8, pages 714-716) is a crude mixture of solamargine, solasonine and their isomeric diglycosides. The extraction process for making BEC involves homogenizing the fruits of *S*. *sodomaeum* in a large volume of acetic acid, filtering off the liquid through muslin followed by precipitation of the glycosides with ammonia (Drugs of today (1990), Vol. 26 No. 1, p. 55-58, cancer letters (1991), Vol. 59, p. 183-192). The yield of the solasodine glycoside mixture is very low (approx. 1%). Moreover the individual process steps are not defined to GMP in terms of scale up, definition of yield, composition and product quality.

WO 03/018604 A discloses the chemical synthesis of solanum glycosides, in particular the synthesis of solamargine as well as beta-monosaccharide intermediates.

Li B. et al., Carbohydrate Research, Elsevier Scientific Publishing Company, Amsterdam, vol. 331. no. 1, pp. 1-7, 9 March 2001 discloses a synthesis of the saponin, polyphyllin D.

Bite P. et al., Acta Chimica Academiae Scientiarum Hungaricae Budapest, vol. 52, no. 1, 1967, pp. 79-82 pertains to the preparation of steroid glycosides.

Paczkowski C et al., Phytochemistry, Vol. 35, No. 6, pp. 1429-1434, 1994 discloses the glycosylation and galactosylation of diosgenin and solasodine by soluble glycosyltransferase(s) from *Solanum Melongena* leaves.

There is a great need for a cost efficient process that provides the antineoplastically active triglycosides such as solamargine and solasonine as well as analogues thereof at high yield with little or no impurities.

Contrary to other steroid ring systems, the steroid skeleton of solasodine contains a very labile nitrogen-containing ring. The same hold true for the steroid ring systems of other alkaloids, notably tomatidine, demissidine or solanidine. These aglycons cannot readily be chemically modified while keeping the steroid skeleton intact. In spite of the fact that the aglycon solasodine is readily available, the prior art does not disclose the synthesis of the solamargine or solasonine using the aglycon as starting material.

The problem underlying the present invention is to provide a cost effective method for the preparation of steroid modified solatrioses such as solamargine and solasonine or analogues thereof in high yields.

Such compounds exhibit cytotoxic activity and may be employed as anticancer agents. Furthermore, such compounds exhibit anti bacterial, anti fungal or anti viral activity.

Accordingly, the present invention provides a method for the preparation of a steroid modified solatriose of general formula (Ib): 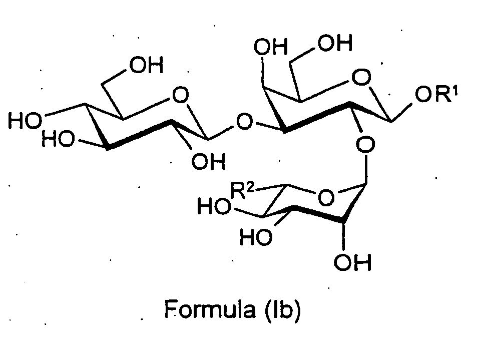 wherein R¹ represents a steroid or a derivative thereof having a hydroxyl group in the 3-position and no further unprotected hydroxyl groups; and R² represents a straight or branched C₁₋₁₄ alkyl group, a C₅₋₁₂ aryl or heteroaryl group optionally substituted by one or more halogen atoms or C₁₋₄ alkyl groups, or a hydroxyl group.

The method comprises the step of: reacting a compound of general formula (IIb): 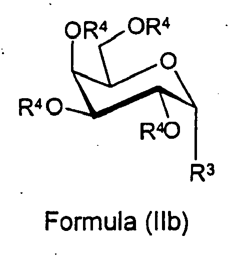 wherein R³ represents a halogen atom, an ethylsulfide or a phenyl sulfide group; and each R⁴ independently represents a benzoyl, substituted benzoyl, whereby the substituents are selected from C₁₋₄ alkyl, groups, halogen atoms and NO₂, acetyl or pivolyl protecting group; with a compound of general formula (III):

HO-R¹ Formula (III)

wherein R¹ is defined as above; to yield a compound of general formula (IVb): 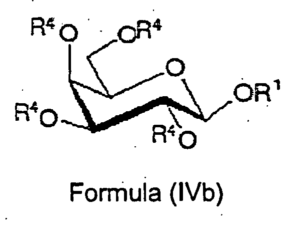 wherein R¹ and R⁴ are defined as above.

The compounds of the above general formula (IVb) is transformed to the desired steroid-modified Solatriose of general formula (Ib).

The present application discloses intermediate compounds , namely:

A compound of general formula (IVb) as defined above;

A compound of general formula (Vb): 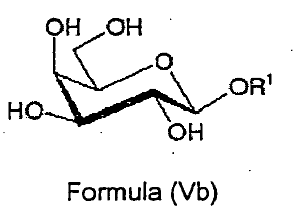 wherein R¹ is defined as above;

The present application provides intermediate compound of general formula (VIb): 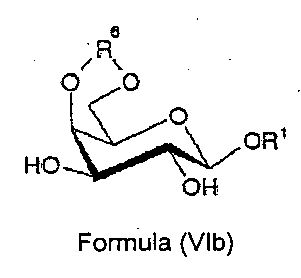 wherein R¹ is as defined above, R⁵ represents a pivolyl protecting group, and R⁶ represents a ketal or acetal type protecting group selected from benzylidene, 4-nitrobenzylidene, 4-methoxybenzylidene or isopropylidene.

Further, a compound of general formula (VIIIb): 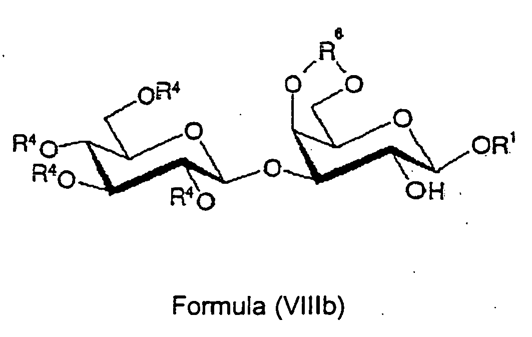 wherein R¹, R², R⁴, R⁵ and R⁶ are as defined above.

A compound of general formula (IXb): 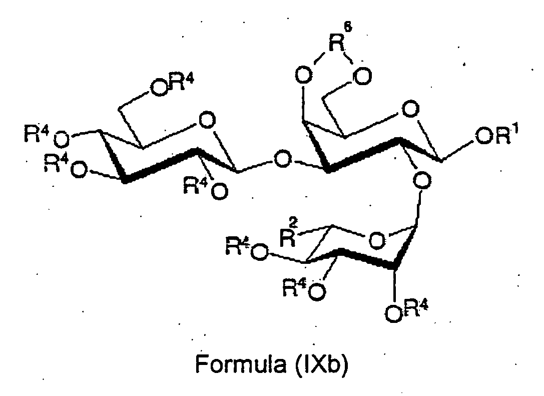 wherein R¹, R², R⁴ and R⁶ are as defined above.

Further embodiments of the present application are described in the dependent claims.

### Detailed description of the invention

In the following, the present invention will be explained in more detail with reference to preferred embodiments.

The steroid residue constituting substituent R¹ is a steroid or a derivative thereof having a hydroxyl group in the 3-position that serves as the α-glycosidic hydroxyl group, which binds the steroid residue to the compound of formula (II) defined above. The steroid residue bears no further unprotected hydroxyl groups and preferably has no further hydroxyl groups at all, in order not to compromise subsequent reaction steps. In a preferred embodiment of the present invention R¹ is selected from a tomatidin-3-yl, demissidin-3-yl, solanidin-3-yl and solasodin-3-yl group.

All of those steroid groups contain a labile nitrogen-containing ring and, therefore, cannot be chemically modified by means of conventional methods. Moreover, all of the above steroid groups represent substituents for cyctotoxic, anti bacterial, anti fungal or anti viral compounds.

In the above general formulae (Ia) and (Ib) each R² independently represents a straight or branched C₁₋₁₄ alkyl group, a C₅₋₁₂ aryl or heteroaryl group optionally substituted by one or more halogen atoms or C₁₋₄ alkyl groups, or a hydroxyl group. In a preferred embodiment R² represents a C₁₋₁₄ alkyl group selected from methyl, ethyl and propyl; an aryl group selected from phenyl, p-methylphenyl and p-chlorophenyl; or an heteroaryl group selected from pyridinyl, pyrimidinyl, furanyl, pyrrolyl, thiophenyl, indolyl, pyrazolyl and imidazolylmethyl; methyl, ethyl and propyl are more preferred.

In a particular preferred embodiment R² represents a methyl group.

The method for preparing a steroid-modified chacotriose of general formula (Ia) not included in the present invention, comprises reacting a compound of general formula (IIa): 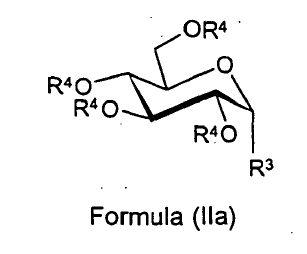 with a compound of general formula (III):

HO-R¹ Formula (III)

to yield a compound of general formula (IVa): (not included in the present invention)

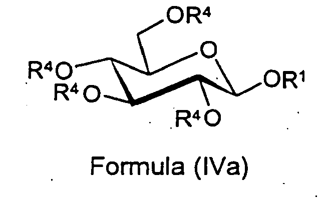

In the above general formula (IIa) R³ represents a halogen atom, an ethylsulfide or a phenyl sulfide group. Preferably, R³ represents a bromine atom or a chlorine atom. Most preferably R³ is a bromine atom. Furthermore, in general formulae (IIa) and (IVa), each R⁴ independently represents a benzoyl, substituted benzoyl, whereby the substituents are selected from C₁₋₄ alkyl groups, halogen atoms and NO₂, acetyl or pivolyl protecting group, preferably a benzoyl or p-toluoyl protecting group, most preferably a benzoyl protecting group.

The above step is preferably conducted in an inert organic solvent such dichloromethane, tetrahydrofuran or dichloromethane. A preferred solvent is dichloromethane.

Preferably the reaction is carried out in the presence of a promoter. Any conventional promoter used in carbohydrate chemistry may be employed. Particular preferred promoters include silver triflate, boron trifluoride diethyl etherate (-10°C), trimethylsilyl triflate bromide, N-iodosuccinimide and dimethyl thiomethyl sulfonium triflate. The most preferred promoter is silver triflate.

The reaction may preferably be carried out under anhydrous conditions in the presence of a water detracting means such as 4Å mol sieves.

In a preferred embodiment the reaction is carried out at low temperature such as 0°C or lower, more preferably -10°C or lower. The most preferred reaction temperature is -20°C.

Subsequently, the above-obtained compound of general formula (lVa) may be further modified as described below.

In the presently described method, the compound of general formula (lVa) is preferrably deprotected by removing substituent R⁴ to obtain a compound of general formula (Va): 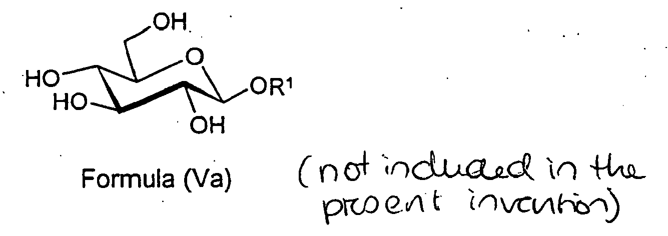 wherein R¹ is defined as above.

Any suitable deprotection condition conventionally employed in the chemistry of protecting groups may be used. Deprotection is preferably carried out in an inert organic solvent such as dichloromethane or tetrahydrofuran in the presence of an alkali metal alkoxide having 1 to 4 carbon atoms and a C₁₋₄ alcohol, or in the presence of water, an alkali metal hydroxide and a C₁₋₄ alcohol. In a particular preferred embodiment deprotection is carried out in dichloromethane in the presence of methanol and sodium methoxide.

The thus obtained compound of general formula (Va) may be selectively protected in 3-OH and 6-OH position using pivolyl chloride in the presence of an amine base to yield compound of general formula (Vla): 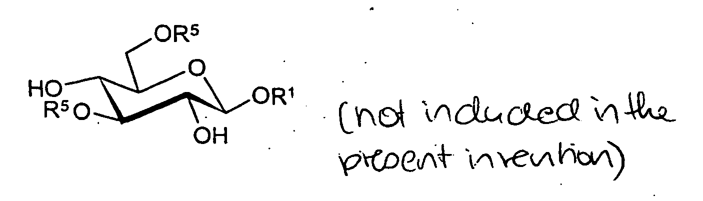 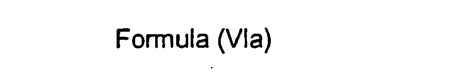 wherein R¹ is as defined above, and R⁵ represents a pivolyl group. Suitable amine bases include pyridine, triethylamine, collidine, or lutidine. A preferred amine base is pyridine.

The reaction may be carried out in an inert organic solvent. Examples of suitable solvents include tetrahydrofuran, dichloroethane, or dimethylformamide.

The compound of formula (VIa) may be then reacted with a compound of general formula (VIIa): 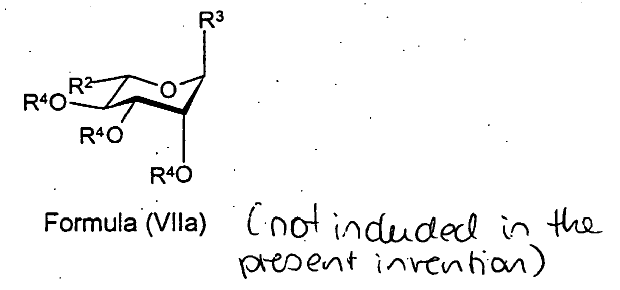 under substantially the same conditions as described above for the preparation of the compound of formula (IVa). In general formula (VIIa) R², R³ and R⁴ are as defined above.

Resulting compound of general formula (Vllla): 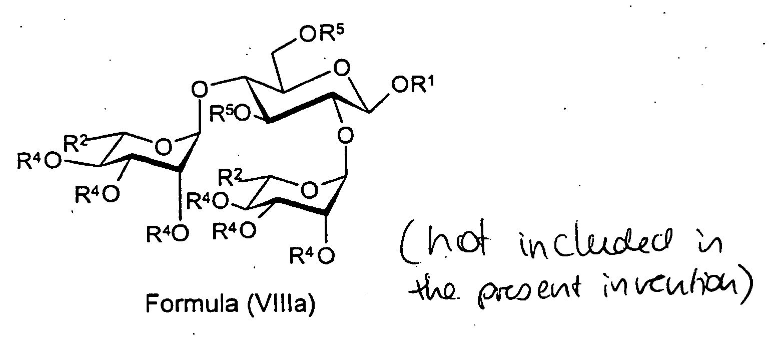 wherein R¹, R², R⁴ and R⁵ are as defined above, may be subsequently deprotected to yield the compound of general formula (Ia) under substantially the same conditions as described above for the preparation of the compound of formula (Va). In a preferred this deprotection step is carried out in tetrahydrofuran in the presence of water, sodium hydroxide and methanol.

The present invention provides a method for preparing a steroid-modified solatriose of general formula (Ib) as claimed in claims 1-18. According to the method for preparing a steroid-modified solatriose of general formula (Ib), galactose is reacted to yield a compound of general formula (IIb): 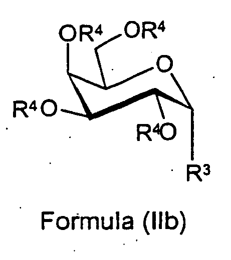 wherein R³ and R⁴ are as defined above.

The preparation of the compound of formula: (IIb) may be carried out using either acetic anhydride, acetyl chloride, benzoyl chloride, benzoic anhydride, or pivolyl chloride in the presence of a base such as, e.g., pyridine, triethylamine, or collidine; to give fully esterified galactose. Esterified-D-galactopyranose may be treated with hydrogenbromide or hydrogenchloride in glacial acetic acid to yield the above compound of general formula (IIb).

In a particularly preferred embodiment galactose is suspended in organic base such as pyridine and cooled to 0°C, to this solution is added dropwise either acetic anhydride, benzoic anhydride or acid chloride. Upon complete addition the solution is warmed to +25°C (room temperature) and stirred for about 16 hours. The reaction is quenched by addition of alcohol. The solution is diluted with organic solvent such as tert-butylmethyl ether, or dichloromethane, or toluene and washed with cold 1N HCl, water, saturated sodium bicarbonate, water and brine then the product is dried over magnesium sulfate and concentrated under reduced pressure to dryness. The product can be used without further purification or it can be recrystallised.

The fully esterified galactopyranose in dry solvent such as dichloromethane is cooled to 0°C under an inert atmosphere. To this solution is added hydrogen bromide in glacial acetic acid, typically 30% HBr content. The solution is allowed to warm to +25°C (room temperature) and stirred for around 16 hours. The solution is diluted with organic solvent such as dichloromethane and then quickly washed with ice cold water, saturated aqueous sodium bicarbonate, and brine. The product is dried over magnesium sulfate filtered and the solvent is removed under reduced pressure. The product is crystallized from petrol (40-60) and diethyl ether.

Furthermore, the method for preparing a steroid-modified solatriose of general formula (Ib) comprises reacting the compound of general formula (IIb) as defined above with a compound of general formula (III) as defined above to yield a compound of general formula (IVb): 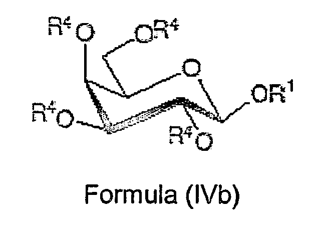 in which R³ and R⁴ are as defined above.

The step for preparing the compound of formula (IVb) is preferably conducted under substantially the same conditions as the reaction for preparing the compound of formula (IVa) above.

Alternatively, the reaction may be carried out by reacting the compound of formula (III) as defined above with intermediate (A): 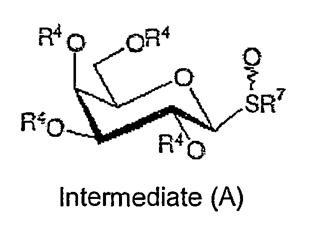 wherein R⁴ is defined above, and R⁷ represents any alkyl or aryl residue, e.g., a straight or branched C₁₋₁₄ alkyl group or a phenyl group optionally substituted with one or more C₁₋₄ alkyl groups; whereby the C₁₋₁₄ alkyl group is preferably selected from methyl, ethyl and propyl and the phenyl group is preferably selected form phenyl, p-methylphenyl and p-chlorophenyl. The reaction can be carried out in a suitable solvent such as dichloromethane or a combination of dichloromethane and an ether such as diethylether. The reaction is preferably carried out in the presence of a promoter as defined above, e.g., triflic anhydride, and a sterically hindered base such as 2,6-lutidine, 2,4,6-collidine or 2,6-di-tertbutyl-4-methyl pyridine, preferably 2,6-di-t-butylpyridine, at low temperature (below -10°C, preferably below -20°C).

In this embodiment, intermediate (A) may be obtained by oxidizing intermediate (B): 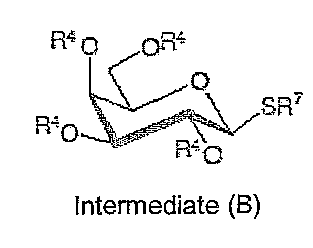 wherein R⁴ and R⁷ are as defined above, to yield the corresponding sulfoxide (i.e., intermediate (A)). Oxidation of intermediate (B) may be effected using a suitable oxidation means, e.g., m-chloroperbenzoic acid. The reaction may be carried out in a solvent such as dichloromethan at low temprature (-20 °C, preferably -40°C).

Intermediate (B) may be formed by the treatment of the compound of formula (IIb) with an alkali metal salt of an alkyl or aryl thiol (R⁷-SH), e.g., the potassium or sodium salt of R⁷-SH, in a suitable solvent such as ethanol or methanol.

Subsequently, the above-obtained compound of general formula (IVb) is deprotected by removing substituent R⁴ to obtain a compound of general formula (Vb): 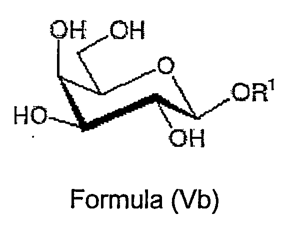 wherein R¹ is defined as above.

Any suitable deprotection condition conventionally employed in the chemistry of protecting groups may be used. In particular, deprotection may preferably be carried out as described above for the preparation of the compound of formula (Va).

The thus obtained compound of general formula (Va) is selectively protected in 4-OH and 6-OH position with a ketal or acetal protecting group using standard conditions to yield a compound of general formula (VIb): 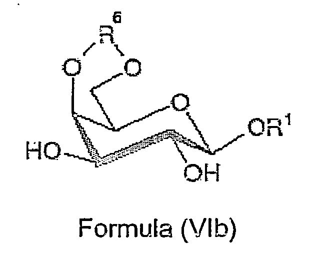 wherein R⁶ represents a ketal or acetal type protecting group selected from benzylidene, 4-nitrobenzylidene, 4-methoxybenzylidene or isopropylidene. In a preferred embodiment R⁷ represents a benzylidene protecting group.

The reaction is preferably carried out in a dipolar aprotic solvent such as dimethyl formamide (DMF) or acetone in the presence of acid catalysts such as p-toluene sulfonic acid or camphorsulfonic acid using a 2,2-dialkyloxypropane or an optionally substituted dialkyloxybenzylidene such as preferably benzaldehyde dimethyl acetal.

Suitable reaction temperatures range from ambient temperature to elevated temperatures. Preferably the reaction is carried out at a temperature of 25°C.

The compound of formula (VIb) may be then reacted with a compound of general formula (Vllb): 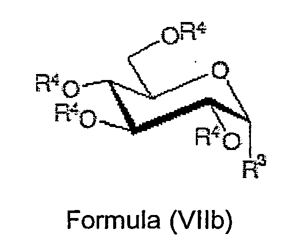 under substantially the same conditions as described above for the preparation of the compound of formula (IVa). In general formula (VIIb) R³ and R⁴ are as defined above. Selective glycosylation at the more reactive 3-position of the galactose may be achieved at reduced temperature such as 0°C or lower, more preferably -10°C or lower. Most preferably the reaction is carried out at about -20°C.

Resulting compound of general formula (VIIIb): 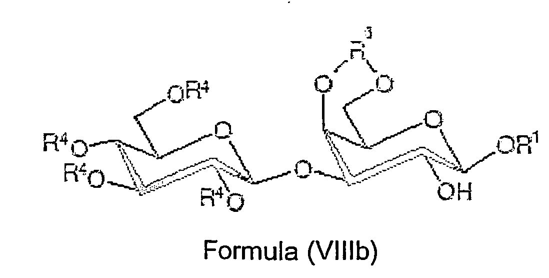 wherein R¹, R⁴ and R⁶ are as defined above, may subsequently be reacted with a compound of formula (VIIa) as defined above under substantially the same conditions as described above for the preparation of the compound of formula (IVa) to yield a compound of general formula (IXb): 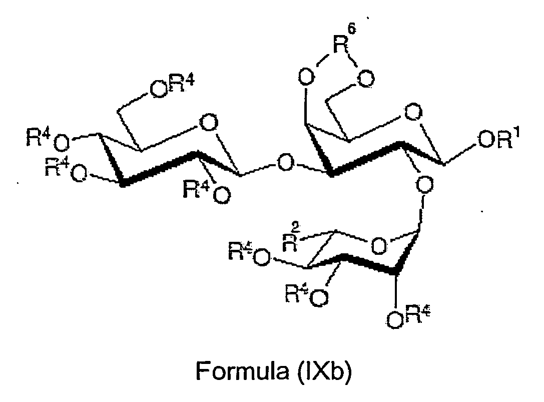 wherein R¹, R², R⁴ and R⁶ are as defined above.

Subsequently, the compound of formula (IXb) may be deprotected to yield the compound of formula (Ib). For example, the ester type protecting group R⁴ may be removed at pH 10-11 under substantially the same conditions as described above for the preparation of the compound of formula (Va). The reaction may then be neutralized by addition of solid carbon dioxide. On the other hand, R⁶ may be removed by using catalytic hydrogenation over palladium on carbon and hydrogen in an appropriate solvent such as ethanol or methanol. It should be understood that the removal of R⁴ and the removal of R⁶ are reversable.

## Claims

1. A method for the preparation of a steroid modified solatriose of general formula (Ib): 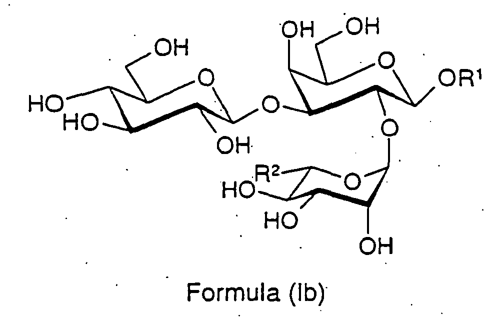 wherein R¹ represents a steroid or a derivative thereof having a hydroxyl group in the 3-position and no further unprotected hydroxyl groups; and R² represents a straight or branched C₁₋₁₄ alkyl group, a C₅₋₁₂ aryl or heteroaryl group optionally substituted by one or more halogen atoms or C₁₋₄ alkyl groups, or a hydroxyl group,
which method comprises the step of:
reacting a compound of general formula (IIb): 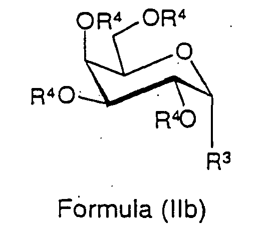
wherein R³ represents a halogen atom, an ethylsulfide or a phenyl sulfide group; and each R⁴ independently represents a benzoyl, substituted benzoyl, whereby the substituents are selected from C₁₋₄ alkyl groups, halogen atoms and NO₂, acetyl or pivolyl protecting group;
with a compound of general formula (III):
HO-R¹ Formula (III)
wherein R¹ is defined as above;
to yield a compound of general formula (IVb): 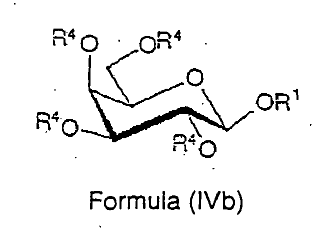 wherein R¹ and R⁴ are defined as above, subsequently deprotecting the compound of general formula (Vb) to yield a compound of general formula (Vb): 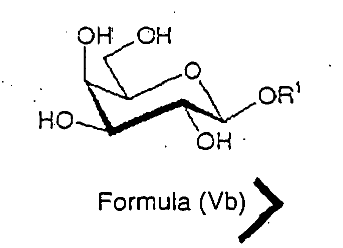 further comprising the step of:
selectively protecting the OH groups in 4- and 6-position of the compound of formula (Vb) with a ketal or acetal protecting type protecting group using standard conditions, to yield a compound of general formula (VIb): 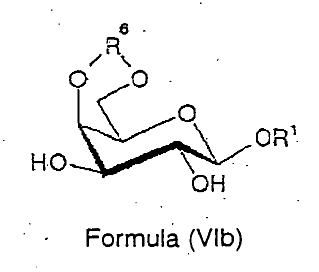 wherein R¹ is as defined above, and R⁶ represents a ketal or acetal type protecting group selected from the group consisting of benzylidene, 4-nitrobenzylidene, 4-methoxybenzylidene and isopropylidene.

2. The method according to claim 1 for preparing a steroid modified solatriose of general formula (lb), further comprising the step of:
reacting a compound of formula (VIb) as defined in claim 1 with a compound of general formula (VIIb): 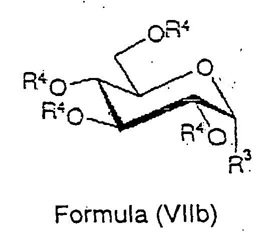
wherein R³ and R⁴ are as defined in claim 1,
to yield a compound general formula (VIIIb): 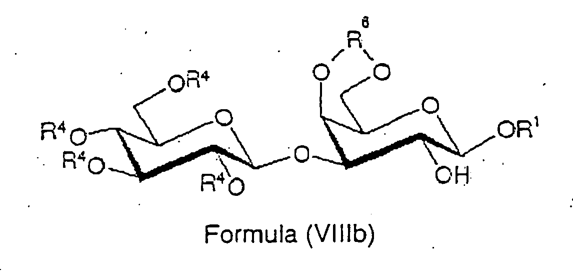 wherein R¹, R⁴ and R⁶ are as defined in claim 1.

3. The method according to any of claims 1 or 2 for preparing a steroid modified solatriose of general formula (Ib), further comprising the step of:
reacting a compound of formula (VIIIb) as defined in claim 2 with a compound of formula (VIIa) 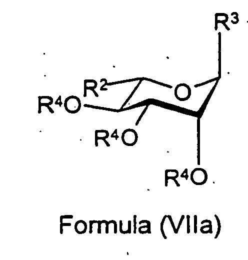 to yield a compound of formula (IXb): 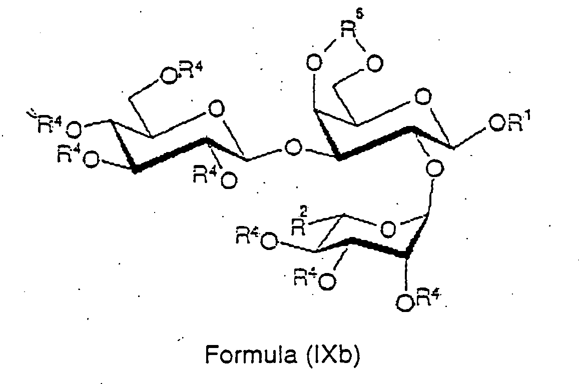
wherein R¹, R², R⁴ and R⁶ are as defined in claim 1.

4. The method according to any of claims 1 to 3 for preparing a steroid modified solatriose of general formula (Ib), further comprising the step of:
deprotecting the compound of formula (IXb) as defined in claim 3 to yield the compound of formula (Ib).

5. The method according to any of the preceding claims, wherein R¹ represents a tomatidin-3-yl, demissidin-3-yl, solanidin-3-yl and solasodin-3-yl group.

6. The method according to any of the preceding claims, wherein R² represents a methyl group.

7. The method according to any of the preceding claims, wherein R³ in the compounds of formulae (IIb) and/or (Vllb) represents a bromine atom.

8. The method according to claim 1, 2 or 3, wherein the reaction is carried out in the presence of a promoter.

9. The method according to claim 8, wherein the promoter is selected from the group consisting of silver triflate, boron trifluoride diethyl etherate, trimethylsilyl triflate bromide, N-iodosuccinimide and dimethyl thiomethyl sulfonium triflate.

10. The method according to claim 9, wherein the promoter is silver triflate.

11. The method according to any of claims 1, 2 or 3, wherein the reaction is carried out under anhydrous conditions in the presence of 4Å mol sieves.

12. The method according to claim 1, wherein deprotection is carried out in dichloromethane or tetrahydrofuran in the presence of a C₁₋₄ alcohol and an alkali metal alkoxide having 1 to 4 carbon atoms.

13. The method according to claim 12, wherein deprotection is carried out in dichloromethane in the presence of methanol and sodium methoxide.

14. The method according to claim 1, wherein deprotection is carried out in dichloromethane or tetrahydrofuran in the presence of water, an alkali metal hydroxide and a C₁₋₄ alcohol.

15. The method according to claim 14, wherein deprotection is carried out in tetrahydrofuran, and wherein the alkali metal hydroxide is sodium hydroxide and the alcohol is methanol.

16. The method according to claim 1 for preparing a steroid modified solatriose of general formula (Ib), wherein R⁴ represents a benzoyl or p-toluolyl protecting group.

17. The method according to any of the preceding claims, wherein reacting a compound of general formula (IIb) with a compound of general formula (III) is carried out in the presence of sterically hindered non-nucleophilic base.

18. The method according to claim 17, wherein the sterically hindered non-nucleophilic base is selected from 2,6-lutidine, 2,4,6-collidine or 2,6-di-tertbutyl-4-methyl pyridine.

19. A compound of general formula (VIIIb) as defined in claim 2; a compound of general formula (VIb) as defined in claim 1, or a compound of general formula (IXb) as defined in claim 3.

## Patentansprüche

1. Verfahren zur Herstellung einer Steroid-modifizierten Solatriose der allgemeinen Formel (Ib): 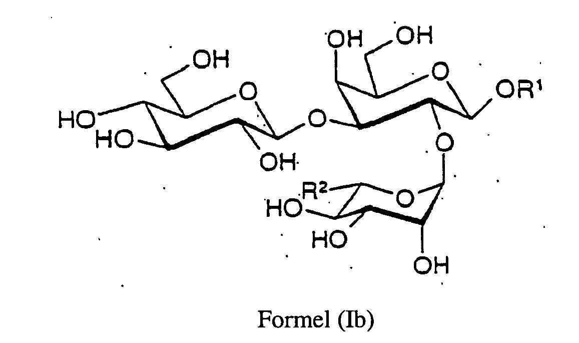 wobei R¹ ein Steroid oder ein Derivat davon mit einem Hydroxylrest an Position 3 und ohne weitere ungeschützte Hydroxylreste darstellt; und R² einen geradkettigen oder verzweigten C₁-C₁₄-Alkylrest, einen C₅-C₁₂-Aryl- oder Heteroarylrest, gegebenenfalls substituiert mit einem oder mehreren Halogenatomen oder C₁-C₄-Alkylresten, oder einen Hydroxylrest darstellt,
wobei das Verfahren den Schritt umfasst:
Umsetzen einer Verbindung der allgemeinen Formel (IIb): 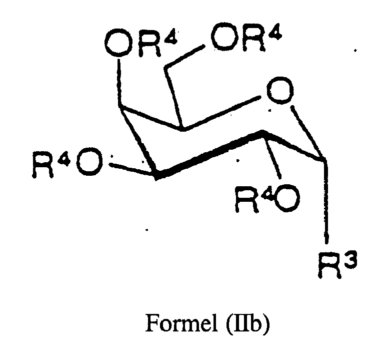
wobei R³ ein Halogenatom, ein Ethylsulfid- oder ein Phenylsulfidrest darstellt; und R⁴ jeweils unabhängig einen Benzoylrest, substituierten Benzoylrest darstellt, wobei die Substituenten ausgewählt sind aus C₁-C₄-Alkylresten, Halogenatomen und NO₂, Acetyl oder Pivolylschutzgruppen;
mit einer Verbindung der allgemeinen Formel (III):
HO-R¹ Formel (III)
wobei R¹ wie vorstehend definiert ist;
so dass sich eine Verbindung der allgemeinen Formel (IVb) ergibt: 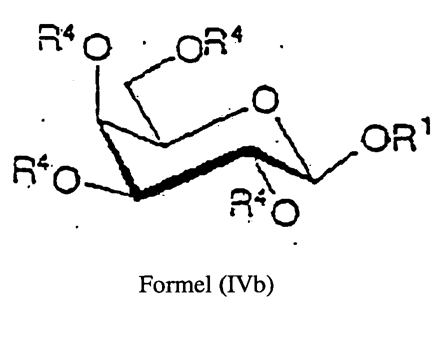 wobei R¹ und R⁴ wie vorstehend definiert sind,
anschließend Entschützen der Verbindung der allgemeinen Formel (IVb), so dass sich eine Verbindung der allgemeinen Formel (Vb) ergibt: 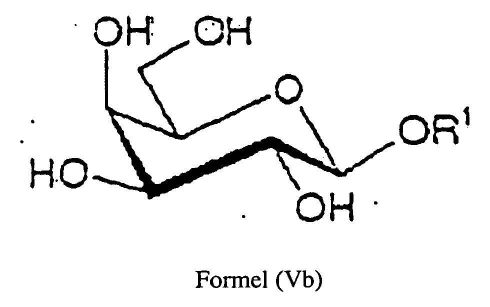 ferner umfassend den Schritt:
selektives Schützen der OH-Gruppen an den Positionen 4 und 6 der Verbindung der Formel (Vb) mit einer Schutzgruppe vom Ketal- oder Acetalschutztyp, unter Verwendung der Standardbedingungen, so dass sich eine Verbindung der allgemeinen Formel (VIb) ergibt: 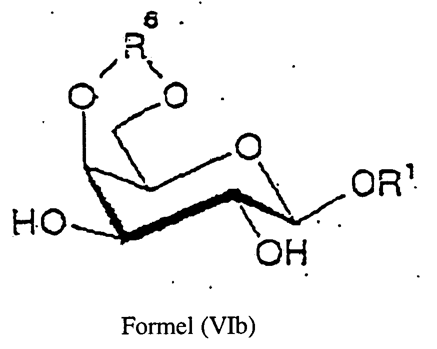
wobei R¹ wie vorstehend definiert ist, und R⁶ eine Schutzgruppe vom Ketal- oder Acetaltyp darstellt, ausgewählt aus Benzyliden, 4-Nitrobenzyliden, 4-Methoxybenzylidene und Isopropyliden.

2. Verfahren gemäß Anspruch 1 zur Herstellung einer Steroid-modifizierten Solatriose der allgemeinen Formel (Ib), ferner umfassend den Schritt:
Umsetzen einer Verbindung der Formel (VIb) wie in Anspruch 1 definiert mit einer Verbindung der allgemeinen Formel (VIIb): 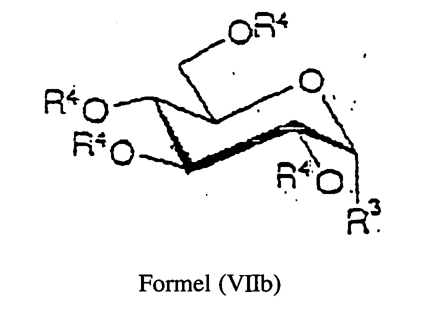
wobei R³ und R⁴ wie in Anspruch 1 definiert sind,
so dass sich eine Verbindung der allgemeinen Formel (VIIIb) ergibt: 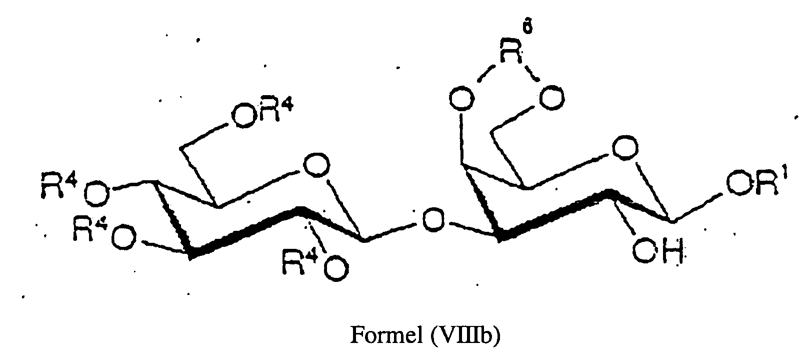 wobei R¹, R⁴ und R⁶ wie in Anspruch 1 definiert sind.

3. Verfahren gemäß einem der Ansprüche 1 oder 2 zur Herstellung einer Steroid-modifizierten Solatriose der allgemeinen Formel (Ib), ferner umfassend den Schritt:
Umsetzen einer Verbindung der Formel (VIIIb) wie in Anspruch 2 definiert mit einer Verbindung der Formel (VIIa) 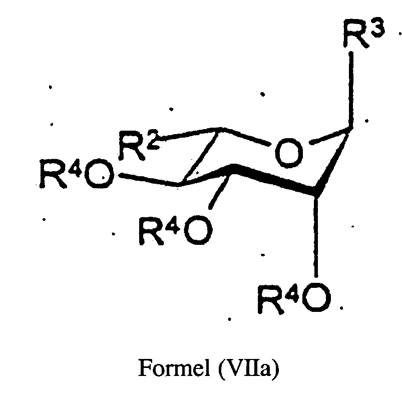 so dass sich eine Verbindung der Formel (IXb) ergibt: 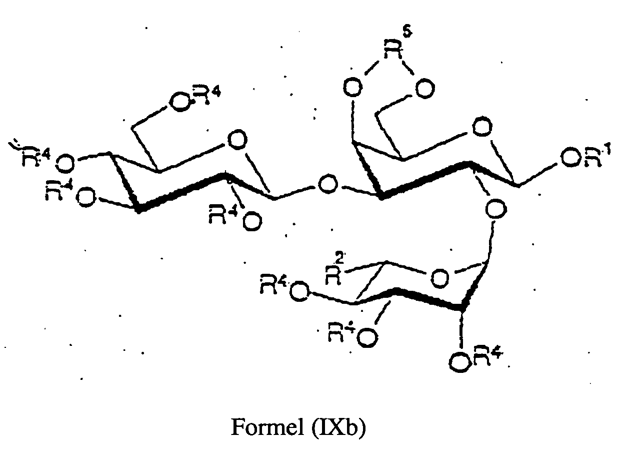
wobei R¹, R², R⁴ und R⁶ wie in Anspruch 1 definiert sind.

4. Verfahren gemäß einem der Ansprüche 1 bis 3 zur Herstellung einer Steroid-modifizierten Solatriose der allgemeinen Formel (Ib), ferner umfassend den Schritt:
Entschützen der Verbindung der Formel (IXb) wie in Anspruch 3 definiert, so dass sich die Verbindung der Formel (Ib) ergibt.

5. Verfahren gemäß einem der vorstehenden Ansprüche, wobei R¹ einen Tomatidin-3-yl-, Demissidin-3-yl-, Solanidin-3-yl- und Solasodin-3-ylrest darstellt.

6. Verfahren gemäß einem der vorstehenden Ansprüche, wobei R² eine Methylgruppe darstellt.

7. Verfahren gemäß einem der vorstehenden Ansprüche, wobei R³ in den Verbindungen der Formeln (IIb) und/oder (VIIb) ein Bromatom darstellt.

8. Verfahren gemäß Anspruch 1, 2 oder 3, wobei die Reaktion in Gegenwart eines Promotors ausgeführt wird.

9. Verfahren gemäß Anspruch 8, wobei der Promotor ausgewählt ist aus Silbertriflat, Bromtrifluoriddiethyletherat, Trimethylsilyltriflatbromid, N-Iodsuccinimid und Dimethylthiomethylsulfoniumtriflat.

10. Verfahren gemäß Anspruch 9, wobei der Promotor Silbertriflat ist.

11. Verfahren gemäß einem der Ansprüche 1, 2 oder 3, wobei die Reaktion unter wasserfreien Bedingungen in Gegenwart von 4 Å Molekularsieben durchgeführt wird.

12. Verfahren gemäß Anspruch 1, wobei das Entschützen in Dichlormethan oder Tetrahydrofuran in Gegenwart eines C₁₋₄-Alkohols und einem Alkalimetalloxids mit 1 bis 4 Kohlenstoffatomen durchgeführt wird.

13. Verfahren gemäß Anspruch 12, wobei das Entschützen in Dichlormethan in Gegenwart von Methanol und Natriummethoxid durchgeführt wird.

14. Verfahren gemäß Anspruch 1, wobei das Entschützen in Dichlormethan oder Tetrahydrofuran in Gegenwart von Wasser, einem Alkalimetallhydroxid und einem C₁₋₄-Alkohol durchgeführt wird.

15. Verfahren gemäß Anspruch 14, wobei das Entschützen in Tetrahydrofuran durchgeführt wird und wobei das Alkalimetallhydroxid Natriumhydroxid ist und der Alkohol Methanol ist.

16. Verfahren gemäß Anspruch 1 zur Herstellung einer Steroid-modifizierten Solatriose der allgemeinen Formel (Ib), wobei R⁴ eine Benzoyl- oder p-Toluolyl-Schutzgruppe darstellt.

17. Verfahren gemäß einem der vorstehenden Ansprüche, wobei das Umsetzen einer Verbindung der allgemeinen Formel (IIb) mit einer Verbindung der allgemeinen Formel (III) in Gegenwart einer sterisch gehinderten nicht nukleophilen Base durchgeführt wird.

18. Verfahren gemäß Anspruch 17, wobei die sterisch gehinderte nicht nukleophile Base ausgewählt ist aus 2,6-Lutidin, 2,4,6-Collidin oder 2,6-Di-tert-butyl-4-methylpyridin.

19. Verbindung der allgemeinen Formel (VIIIb) wie in Anspruch 2 definiert, eine Verbindung der allgemeinen Formel (VIb) wie in Anspruch 1 definiert oder eine Verbindung der allgemeinen Formel (IXb) wie in Anspruch 3 definiert.

## Revendications

1. Procédé de préparation d'un solatriose modifié par un stéroïde de formule générale (Ib) : 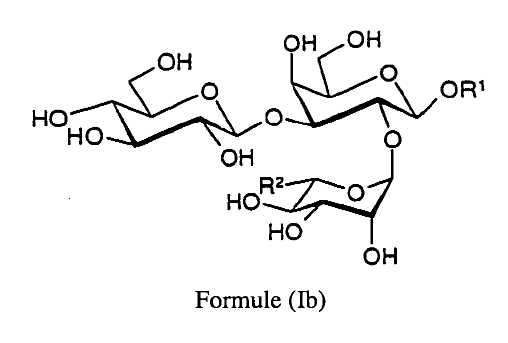 dans laquelle R¹ représente un stéroïde ou un dérivé de celui-ci ayant un groupe hydroxyle en position 3 et pas d'autres groupes hydroxyles non protégés ; et R² représente un groupe alkyle en C₁-C₁₄ à chaîne linéaire ou ramifiée, un groupe aryle ou hétéroaryle en C₅-C₁₂ éventuellement substitué par un ou plusieurs atomes d'halogène ou groupes alkyle en C₁-C₄, ou un groupe hydroxyle,
lequel procédé comprend les étapes de :
faire réagir un composé de formule générale (IIb) : 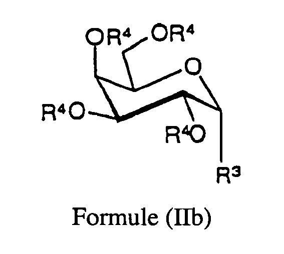
dans laquelle R³ représente un atome d'halogène, un groupe éthylsulfide ou un groupe phénylsulfide ; et chaque R⁴ représente indépendamment un groupe protecteur benzoyle, benzoyle substitué, où les substituants sont choisis parmi les groupes alkyle en C₁-C₄, les atomes d'halogène et NO₂, acétyle ou pivolyle ;
avec un composé de formule générale (III) :
HO-R¹ Formule (III)
dans laquelle R¹ est défini comme ci-dessus ;
pour donner un composé de formule générale (IVb) : 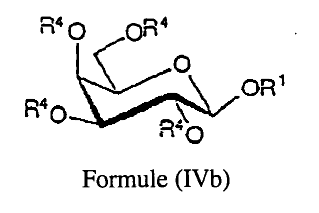 dans laquelle R¹ et R⁴ sont tels que définis ci-dessus,
puis déprotéger le composé de formule générale (IVb) pour donner un composé de formule générale (Vb) : 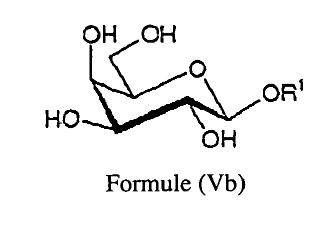 comprenant en outre l'étape de :
protéger sélectivement les groupes OH en positions 4 et 6 du composé de formule (Vb) avec un groupe protecteur de type cétal ou acétal en utilisant des conditions standard, pour donner un composé de formule générale (VIb) : 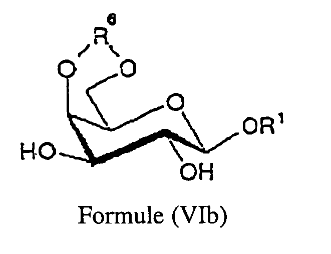
dans laquelle R¹ est tel que défini ci-dessus, et R⁶ représente un groupe protecteur de type cétal ou acétal choisi dans le groupe constitué par le benzylidène, le 4-nitrobenzylidène, le 4-méthoxybenzylidène et l'isopropylidène.

2. Procédé selon la revendication 1 de préparation d'un solatriose modifié par un stéroïde de formule générale (Ib), comprenant en outre l'étape de :
faire réagir un composé de formule (VIb) tel que défini selon la revendication 1, avec un composé de formule générale (VIIb) : 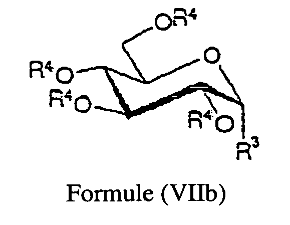
dans laquelle R³ et R⁴ sont tels que définis selon la revendication 1,
pour donner un composé de formule générale (VIIIb) : 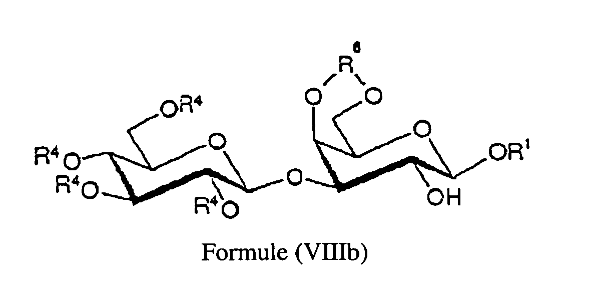 dans laquelle R¹, R⁴ et R⁶ sont tels que définis selon la revendication 1.

3. Procédé selon la revendication 1 ou 2 de préparation d'un solatriose modifié par un stéroïde de formule générale (Ib), comprenant en outre l'étape de :
faire réagir un composé de formule (VIIIb) tel que défini selon la revendication 2, avec un composé de formule générale (VIIa) : 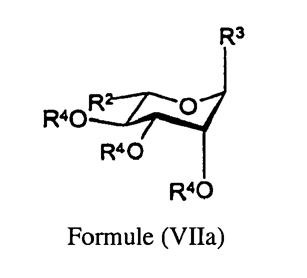
pour donner un composé de formule générale (IXb) : 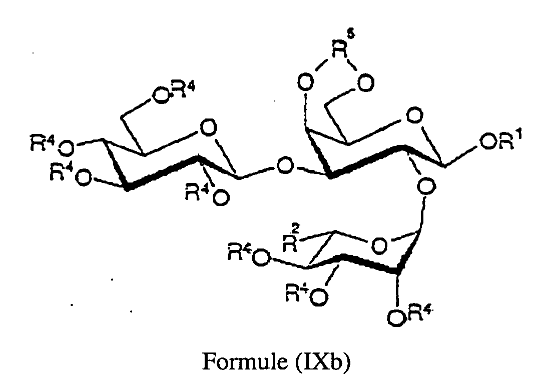
dans laquelle R¹, R², R⁴ et R⁶ sont tels que définis selon la revendication 1.

4. Procédé selon l'une quelconque des revendications 1 à 3 de préparation d'un solatriose modifié par un stéroïde de formule générale (Ib), comprenant en outre l'étape de :
déprotéger le composé de formule (IXb) tel que défini selon la revendication 3 pour donner le composé de formule (Ib).

5. Procédé selon l'une quelconque des revendications précédentes, dans lequel R¹ représente un groupe tomatidin-3-yle, démissidin-3-yle, solanidin-3-yle et solasodin-3-yle.

6. Procédé selon l'une quelconque des revendications précédentes, dans lequel R² représente un groupe méthyle.

7. Procédé selon l'une quelconque des revendications précédentes, dans lequel R³ dans les composés de formule (IIb) et/ou (VIIb) représente un atome de brome.

8. Procédé selon la revendication 1, 2 ou 3, dans lequel la réaction est réalisée en présence d'un promoteur.

9. Procédé selon la revendication 8, dans lequel le promoteur est choisi dans le groupe constitué par le triflate d'argent, l'éthérate diéthylique de trifluorure de bore, le bromure de triméthylsilyltriflate, le N-iodosuccinimide et le triflate de diméthylthiométhylsulfonium.

10. Procédé selon la revendication 9, dans lequel le promoteur est le triflate d'argent.

11. Procédé selon l'une quelconque des revendications 1, 2 ou 3, dans lequel la réaction est réalisée dans des conditions anhydres en présence de tamis moléculaires de 4Å.

12. Procédé selon la revendication 1, dans lequel la déprotection est réalisée dans du dichlorométhane ou du tétrahydrofurane en présence d'un alcool en C₁-C₄ et d'un alcoxyde de métal alcalin ayant de 1 à 4 atomes de carbone.

13. Procédé selon la revendication 12, dans lequel la déprotection est réalisée dans du dichlorométhane en présence de méthanol et de méthoxyde de sodium.

14. Procédé selon la revendication 1, dans lequel la déprotection est réalisée dans du dichlorométhane ou du tétrahydrofurane en présence d'eau, d'un hydroxyde de métal alcalin et d'un alcool en C₁-C₄.

15. Procédé selon la revendication 14, dans lequel la déprotection est réalisée dans du tétrahydrofurane, et dans lequel l'hydroxyde de métal alcalin est l'hydroxyde de sodium et l'alcool est le méthanol.

16. Procédé selon la revendication 1 de préparation d'un solatriose modifié par un stéroïde de formule générale (Ib), dans lequel R⁴ représente un groupe protecteur benzoyle ou p-toluolyle.

17. Procédé selon l'une quelconque des revendications précédentes, dans lequel la réaction d'un composé de formule générale (IIb) avec un composé de formule générale (III) est réalisée en présence d'une base non nucléophile stériquement encombrée.

18. Procédé selon la revendication 17, dans lequel la base non nucléophile stériquement encombrée est choisie parmi la 2,6-lutidine, la 2,4,6-collidine ou la 2,6-di-tertbutyl-4-méthylpyridine.

19. Composé de formule générale (VIIIb) tel que défini dans la revendication 2 ;
composé de formule générale (VIb) tel que défini dans la revendication 1 ; ou
composé de formule générale (IXb) tel que défini dans la revendication 3.
